(19) Europäisches Patentamt
European Patent Office
Office européen des brevets

(11) Numéro de publication: **0 392 258 B1**

(12) # FASCICULE DE BREVET EUROPEEN

(45) Date de publication de fascicule du brevet: **19.04.95**

(21) Numéro de dépôt: **90105878.4**

(22) Date de dépôt: **28.03.90**

(51) Int. Cl.⁶: **C07C 47/277**, C07C 47/232, A61K 7/46, C11B 9/00, A01N 31/04, A01N 43/40, C07C 45/74, C07C 45/62, C07C 45/67, C07C 43/315, C07C 43/303

(54) **Aldéhydes aromatiques nouveaux, leurs dérivés et leur utilisation à titre d'ingrédients parfumants, herbicides et fongicides.**

(30) Priorité: **12.04.89 CH 1379/89**

(43) Date de publication de la demande:
**17.10.90 Bulletin 90/42**

(45) Mention de la délivrance du brevet:
**19.04.95 Bulletin 95/16**

(84) Etats contractants désignés:
**AT BE CH DE DK ES FR GB GR IT LI LU NL SE**

(56) Documents cités:
**CH-A- 133 680**
**FR-A- 1 460 826**

(73) Titulaire: **FIRMENICH SA**
**1, route des Jeunes**
**CH-1211 Genève 8 (CH)**

(72) Inventeur: **Naef, Ferdinand**
**30, Chemin Vert**
**CH-1227 Carouge (CH)**
Inventeur: **Delay, François**
**19, rue de la Fontenette**
**CH-1227 Carouge (CH)**
Inventeur: **Uijttewaal, Arnoldus**
**Le Joran,**
**Challex**
**F-01630 St-Genis-Pouilly (FR)**

(74) Mandataire: **Salvadori, Giuseppe, Dr.**
**c/o Firmenich S.A.**
**Case Postale 239**
**CH-1211 Genève 8 (CH)**

EP 0 392 258 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a trait au domaine de la parfumerie. L'invention concerne plus particulièrement des aldéhydes aromatiques nouveaux et leurs dérivés définis à l'aide de la formule générale

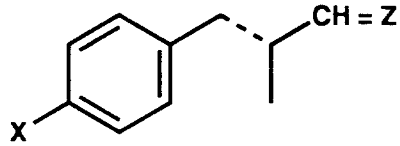

(I)

pouvant posséder une simple ou double liaison dans la position indiquée par les pointillés et dans laquelle le symbole X représente un radical monovalent de formule

a. 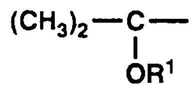

ou, lorsque les pointillés représentent une liaison simple, de formule

b. 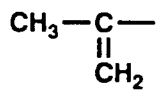

et le symbole Z sert à définir un atome d'oxygène ou deux restes $R^2O$, $R^1$ et $R^2$ représentant des radicaux alkyle de 1 à 3 atomes de carbone.

L'aldéhyde p-tert-butyl-$\alpha$-méthyl-hydrocinnamique, tout comme son homologue inférieur, l'aldéhyde cyclamen, a rencontré depuis sa découverte [voir le brevet US 2'875'131] un succès grandissant en parfumerie. Commercialisé sous différentes dénominations, cet aldéhyde a trouvé un emploi particulier dans la création de compositions à caractère floral où il s'accorde harmonieusement avec des composés de type boisé et musqué.

Vu l'intérêt suscité par ce composé, plusieurs groupes de recherche se sont attelés à en réaliser la synthèse et certains analogues structuraux ont été proposés à titre de remplacement; à ce jour toutefois sans succès. C'est de manière inattendue que nous avons découvert que les composés de formule (I) possédaient des propriétés odorantes exceptionnelles et que, de ce fait, ils pouvaient trouver un emploi étendu en parfumerie.

Leurs caractères odorants, tout en s'apparentant à ceux de l'aldéhyde connu, de par leur connotation florale, se distinguent de ceux-ci par une note de type fleur blanche, muguet. Ils possèdent en outre un côté plus doux, plus fleuri et moins vert-aqueux, ainsi qu'un aspect velouté élégant. La note florale est beaucoup plus fraîche et leur caractère fleur blanche est moins agressif que celui propre aux composés connus; de ce fait, leur emploi est plus étendu.

Les caractéristiques les plus prononcées se retrouvent dans le 3-[4-(1-méthoxy-1-méthyléthyl)-1-phényl]-2-méthylpropanal.

Le 3-(4-isopropényl-1-phényl)-2-méthylpropanal est par contre caractérisé par une note plus verte, type vert-melon. Il rappelle l'aldéhyde cyclamen, étant toutefois moins acide et moins aldéhydé que celui-ci et possédant un côté hespéridé qui ne se retrouve pas dans le composé connu. Ce composé de l'invention possède en outre un aspect légèrement farineux qui n'est pas sans rappeler l'essence de néroli.

Or, rien de laissait présager dans l'état de la technique que de tels composés pouvaient en effet posséder des propriétés particulières et, par ailleurs, aucune indication n'y était fournie quant à l'opportunité de les synthétiser.

De par leurs propriétés odorantes, les composés de l'invention peuvent être employés pour le parfumage d'articles divers et pour la préparation de bases parfumantes et de parfums. Leur emploi peut être fort général. Parmi les articles qui peuvent être parfumés à l'aide des aldéhydes aromatiques (I), il convient de citer à titre d'exemple les savons, les détergents, solides ou liquides, ioniques, anioniques ou zwitterioniques ou non-ioniques, les adoucissants textiles, les produits d'entretien ou encore les cosmétiques, les shampoings et les désodorisants corporels. Employés en tant qu'adjuvants actifs dans des bases polymériques ou résines variées, les aldéhydes aromatiques de l'invention peuvent servir comme principes actifs pour des dispositifs désodorisants ou assainissants d'air ambiant ou d'enceintes fermées. Les proportions dans lesquelles les composés de l'invention peuvent développer les caractères odorants désirés peuvent bien entendu varier dans une gamme de valeurs très étendue. L'homme de l'art sait par expérience que de telles valeurs dépendent de l'effet particulier recherché ainsi que de la nature des produits que l'on désire parfumer. On sait également que ces valeurs sont fonction de la nature des autres constituants dans une composition donnée lorsque les aldéhydes (I) sont utilisés en tant qu'ingrédients dans une base parfumante ou dans un concentré en mélange avec d'autres coingrédients parfumants, des solvants ou des adjuvants usuels.

Les coingrédients avec lesquels les composés de l'invention peuvent être employés appartiennent à des classes chimiques variées, par exemple aldéhydes, esters, éthers et alcools; ils peuvent être naturels ou synthétiques. La mention spécifique de tels coingrédients est ici superflue. L'état de la technique est en effet riche en exemples et l'homme de l'art pourra choisir parmi les ingrédients connus ceux qui se prêtent le mieux à satisfaire son objectif de création. Une référence particulière est faite ici à l'ouvrage de S. Arctander, Perfume and Flavor Chemicals, Montclair, N.J. (USA), 1969.

Compte tenu de leur puissance olfactive, les aldéhydes (I) peuvent être employés à des concentrations pouvant varier entre des valeurs de 1 jusqu'à 20 ou 25% en poids par rapport au poids de la composition dans laquelle ils sont incorporés.

Bien entendu, lorsque lesdits composés sont employés pour le parfumage d'articles de consommation, tels ceux énumérés plus haut, ces valeurs de concentrations sont inférieures et peuvent être de l'ordre de 0,5-1,0%.

Les valeurs des concentrations indiquées doivent être interprétées de manière non restrictive et des valeurs autres que celles-ci peuvent être employées lorsqu'on désire obtenir des effets particuliers.

Comme indiqué plus haut, les composés de l'invention sont nouveaux. Ils peuvent être synthétisés à l'aide d'un procédé faisant appel à des méthodes analogues à celles qui sont mentionnées dans l'art antérieur à partir d'un produit disponible commercialement, à savoir le 4-(1-méthoxy-1-méthyléthyl)-benzaldéhyde [origine : BASF AG, Ludwigshafen/Rh. (REA); voir demande de brevet européen publiée sous le numéro 275 489].

Le procédé consiste en ce qu'on

a. additionne le propanal à l'aldéhyde 8-méthoxycuminique pour donner le 3-[4-(1-méthoxy-1-méthyléthyl)-1-phényl]-2-méthyl-2-propénal;

b. réduit ledit composé ainsi formé par hydrogénation catalytique en présence d'un catalyseur métallique pour fournir le 3-[4-(1-méthoxy-1-méthyléthyl)-1-phényl]-2-méthylpropanal;

c. traite le composé ainsi formé avec un agent acide fort pour fournir le 3-(4-isopropényl-1-phényl)-2-méthylpropanal et, le cas échéant,

d. transforme les deux aldéhydes obtenus suivant les lettres b. ou c. ci-dessus en leurs dérivés acétals correspondants au moyen des méthodes usuelles.

La première étape du procédé s'effectue en milieu basique, de préférence en présence d'une base forte tel un hydroxyde alcalin comme l'hydroxyde de potassium en solution alcoolique, le méthanol en particulier.

Quant à la réduction, elle est effectuée par hydrogénation catalytique en présence d'un catalyseur métallique usuel connu pour promouvoir la réduction d'une double liaison éthylénique. A titre préférentiel, on emploie le palladium, notamment le palladium sur un support solide, par exemple sur alumine. On a ainsi obtenu le 3-[4-(1-méthoxy-1-méthyléthyl)-1-phényl]-2-méthylpropanal.

Par acidification de ce produit, on a pu obtenir le composé déméthoxylé correspondant, ou 3-(4-isopropényl-1-phényl)-2-méthylpropanal.

La transformation de ce dernier composé en ses dérivés acétals correspondants, ainsi que la formation des acétals du 3-[-4-(1-méthoxy-1-méthyléthyl)-1-phényl]-2-méthylpropanal, sont effectuées suivant des méthodes analogues à celles connues dans l'art. Par exemple, le traitement desdits aldéhydes avec un orthoformate de trialkyl dans un milieu constitué par un alcool aliphatique inférieur donne les acétals désirés avec un rendement quantitatif. Des exemples spécifiques de préparation seront fournis plus loin.

Les composés de l'invention de formule (I) trouvent un emploi non seulement en tant qu'ingrédients parfumants mais également en tant que produits de départ pour la préparation de produits finaux à actions fongicides ou herbicides.

La présente invention a également trait à cette utilisation particulière ainsi qu'aux produits actifs qui en résultent. Il s'agit en effet de composés hétérocycliques azotés nouveaux de structure

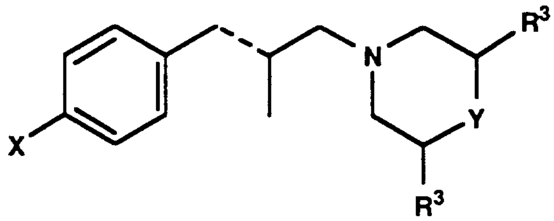

(II)

dans laquelle X et les pointillés sont définis comme indiqué pour la formule (I),. le symbole Y désigne un reste méthylène ou un atome d'oxygène et $R^3$ représente un atome d'hydrogène ou un groupe méthyle. A titre de composés particuliers définis par la formule (II), il convient de citer les composés suivants :
N-{3-[4-(1-méthoxy-1-méthyléthyl)-1-phényl]-2-méthylpropyl}-morpholine,
N-{3-[4-(1-méthoxy-1-méthyléthyl)-1-phényl]-2-méthylpropyl}-pipéridine,
N-{3-[4-(1-méthoxy-1-méthyléthyl)-1-phényl]-2-méthylpropyl}-2,6-diméthylmorpholine,
N-[-3-(4-isopropényl-1-phényl)-2-méthylpropyl]-pipéridine, et
N-[-3-(4-isopropényl-1-phényl)-2-méthylpropyl]-morpholine.

Les composés (II) ont montré une bonne activité fongicide préventive contre la rouille des pois et du froment ainsi que contre le mildiou poudreux du raisin et du froment. Ces composés sont également actifs contre le mildiou poudreux de l'orge. Aucun dommage sur les plantes n'a été observé lors du traitement à l'aide des composés actifs lorsque ceux-ci ont été utilisés dans les limites des dosages expérimentaux, à savoir égaux ou inférieurs à 125 mg/l. Les composés actifs (II) peuvent être utilisés à des concentrations pouvant varier entre 5 et 500 mg/l de solution active. Leur concentration lors des applications expérimentales directes sur les champs de céréales était comprise entre 100 et 2500 mg de substance active par hectare.

Les composés de formule (II) sont préparés à partir des aldéhydes de formule (I) suivant des méthodes de synthèse analogues à celles décrites dans la littérature par addition des composés de formule

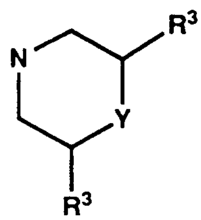

(III)

dans laquelle le symbole Y et le substituant $R^3$ ont le sens donné précédemment. La méthode particulière de préparation desdits composés (II) sera décrite dans le détail dans les exemples donnés plus loin.

L'invention est illustrée par, mais non limitée aux exemples suivants dans lesquels les températures sont indiquées en degrés centigrades et les abréviations ont le sens usuel dans l'art.

Exemple 1

Préparation de 3-[4-(1-méthoxy-1-méthyléthyl)-1-phényl]-2-méthylpropanal

a. (E)-3-[4-(1-Méthoxy-1-méthyléthyl)-1-phényl]-2-méthyl-2-propénal

On ajouté lentement 552g (9,5 mole) de propanal à un mélange agité de 1000 g (5,6 mole) de 8-méthoxycuminaldéhyde (origine : BASF AG, Ludwigshafen/Rh., RFA), 172 g de KOH aqueux à 45% et 3360 g de méthanol. La réaction est légèrement exothermique et, à la fin de l'addition, le mélange atteint 35°. Le mélange a été maintenu sous agitation pendant encore 1 h, puis on a ajusté le pH à 7,5 avec de l'acide acétique et le méthanol a été distillé à pression ordinaire. Après refroidissement à 60°, on a ajouté 810 g d'eau, puis les sels précipités ont été dissous par agitation pendant 30 mn. La phase organique séparée a été distillée sur une petite colonne Vigreux de 15 cm à une pression de 0,1 mbar. On a ainsi obtenu 906 g (4,15 mole) de l'aldéhyde désiré sous forme d'un liquide jaunâtre dont l'Eb. est de 110-114° (rend. 74%).

SM : $M^+$ = 218(3); m/z : 203(65), 186(34), 171(11), 145(100), 128(37), 115(72), 103(9), 91(26), 77(13), 63(9), 51(8), 43(16),

| | |
|---|---|
| $^{1}H$ RMN(360MHz) : | 1,56(6H,s); 2,11(3H,s); 3,11(3H,s); 7,27(1H, s large); 7,50 et 7,54(4H,2d); 9,59-(1H,s) δ ppm; |
| $^{13}C$ RMN : | 10,9(q); 27,8(2q); 50,7(q); 76,8(s); 126,2(2d); 130,1(2d); 133,8(s); 138,2(s); 148,0-(s); 149,5(d); 195,6(d) δ ppm; |
| IR : | 1660, 1620 et 1600 $cm^{-1}$. |

b. 3-[4-(1-Méthoxy-1-méthyléthyl)-1-phényl]-2-méthylpropanal

160 g (0,733 mole) de l'aldéhyde, préparé suivant la méthode décrite sous letter a. ci-dessus, 1 g de Pd sur alumine à 5%, 0,1 g d'acétate de potassium et 2 g d'eau ont été introduits dans un autoclave de 500 cc. On a ensuite effectué une hydrogénation de ce mélange à 120° sous 20 bars en 3 h. Après refroidissement et filtration, le produit brut (155 g) a été distillé sur une colonne remplie d'hélices en verre (longueur 10 cm). On a ainsi obtenu 145 g (0,66 moles) de l'aldéhyde désiré sous forme d'un liquide incolore ayant Eb. 82-5°/0,1 mbar (rend. 90%).

| | |
|---|---|
| SM : | $M^+$ = 200(1); m/z : 205(100), 189(16), 161(4), 148(47), 131(98), 115(38), 105(23), 91(39), 73(18), 43(13); |
| $^{1}H$ RMN(360MHz) : | 0,92(3H,d,$^{3}J$ = 7,2); 1,32(6H,s); 1,74(1H,s large); 2,41(1H,dd, $^{2}J$ = 11,9,$^{3}J$ = 7,6); 2,75(dd,$^{2}J$ = 11,9,$^{3}J$ = 6,9); 3,06(3H,s); 3,49(2H,m large); 7,14 et 7,32(4H,2d) δ ppm; |
| $^{13}C$ RMN : | 16,6(q); 27,9(2q); 37,8(d); 39,3(t); 50,6(q); 67,5(t); 76,7(s); 125,8(2d); 129,0(2d); 139,3(s); 143,4(s) δ ppm; |
| IR : | 3400$cm^{-1}$. |

Exemple 2

Préparation de 3-(4-isopropényl-1-phényl)-2-méthylpropanal

55 g (0,25 moles) de 3-[4-(1-méthoxy-1-méthyléthyl)-1-phényl]-2-méthylpropanal ont été versés lentement dans 660 ml d'une solution à 10% d'acide sulfurique préchauffée à 95° et traversée par un débit de vapeur de 3 kg/h. A la fin de l'entraînement, les eaux ont été extraites à l'éther diéthylique et la phase organique a été séchée et évaporée. On a ainsi obtenu 45 g de produit brut que l'on a distillé sur une colonne à hélices de 10 cm. On a ainsi obtenu 40 g d'un liquide incolore d'une pureté de 98% (rend. 85%). Eb. 127-130°/0,1 mbar.

| | |
|---|---|
| SM : | $M^+$ = 188(22); m/z : 173(3), 155(2), 145(7), 131(100), 115(26), 103(4), 91(33), 77-(8), 65(5), 51(4), 41(5); |
| $^{1}H$ RMN(360MHz) : | 1,10(3H,d,$^{3}J$ = 7,2); 2,14(3H,s); 2,61(1H,dd,$^{2}J$ = 11,8,$^{3}J$ = 7,8);2,66(1H,m); 3,08-(1H,dd,$^{2}J$ = 11,8,$^{3}J$ = 7,0); 5,06(1H,s); 5,35(1H,s); 7,13 et 7,40(4H,2d) δ ppm; |
| $^{13}C$ RMN : | 13,3(q); 21,8(q); 36,4(t); 48,0(d); 112,1(t); 125,7(2d); 128,9(2d); 138,1(s); 139,7(s); 142,9(s); 204,1(d) δ ppm; |
| IR : | 1715 et 1620 $cm^{-1}$. |

Exemple 3

3-[4-(1-Méthoxy-1-méthyléthyl)-1-phényl]-2-méthylpropionaldéhyde diméthylacétal

Un mélange de 2,03 g (9,23 mmole) de 3-[-4-(1-méthoxy-1-méthyléthyl)-1-phényl]-2-méthylpropanal, 1,09 g (10,3 mmole) d'orthoformate de triméthyl, 20 ml de méthanol anhydre et 20 mg de chlorure d'ammonium a été maintenu sous agitation dans un réacteur à fond rond pendant 18 h à température ambiante. Le mélange de réaction a été ensuite versé dans une solution aqueuse à 5% de bicarbonate de sodium et extrait deux fois avec de l'éther. Les extraits organiques combinés ont été lavés deux fois avec une solution aqueuse concentrée de chlorure de sodium puis concentrés dans un évaporateur rotatif et distillés sur résidu pour fournir une fraction de 2,33 g (rend. 95%) de l'acétal désiré ayant Eb. 130° (température du bain)/0,1 mbar.

| | |
|---|---|
| SM : | $M^+$ = 266(1); m/z : 251(6), 234(16), 219(46), 202(45), 187(39), 155(25), 131(32), 75(100), 43(18); |
| $^{1}H$ RMN(360MHz) : | 0,85(3H,d,J = 6,5); 1,52(6H,s); 3,06(3H,s); 3,8 et 4,0(6H,s); 4,08(1H,d,J = 6,5); 7,14 et 7,32(4H,2d,J = 8) δ ppm. |

En suivant la même procédure que celle indiquée ci-dessus, on a obtenu les acétals suivants :

| produit final | produit de départ | réactif |
|---|---|---|
| (a) | (b) | triéthylformiate |
| (c) | (d) | triméthylformiate |
| (e) | (d) | triéthylformiate |

(a) = 3-[4-(1-méthoxy-1-méthyléthyl)-1-phényl]-2-méthylpropionaldéhyde diéthylacétal

(b) = 3-[4-(1-méthoxy-1-méthyléthyl)-1-phényl]-2-méthylpropanal

(c) = 3-(4-isopropényl-1-phényl)-2-méthylpropionaldéhyde diméthylacétal

(d) = 3-(4-isopropényl-1-phényl)-2-méthylpropanal

(e) = 3-(4-isopropényl-1-phényl)-2-méthylpropionaldéhyde diéthylacétal

(a):

SM : $M^+$ = 294(0) ; m/z : 279(1),248(12), 233(25), 216(34), 131(46), 103(100), 75(41), 43(39);

$^1$H RMN(360MHz) : 0,86(3H,d,J = 6,5); 1,22(6H,t,J = 7); 1,52(6H,s); 3,06(3H,s); 3,54 et 3,7(4H,2m); 4,22(1H,d,J = 6,5); 7,13 et 7,32(4H,2d,J = 8) δ ppm.

(c) :

SM : $M^+$ = 234(2); m/z : 202(38), 187(37), 155(21), 131(45), 115(22), 91(21), 75(100);

$^1$H RMN(360MHz) : 0,85(3H,d,J = 6,5); 2,14(3H,s); 3,38 et 3,39(6H,2s); 4,06(1H, d,J = 6,5); 5,04 et 5,36(2H,2s); 7,12 et 7,38(4H,2d,J = 8) δ ppm.

(e) :

SM : $M^+$ = 262(1); m/z : 216(63), 131(98), 103(100), 75(75), 43(95);

$^1$H RMN(360MHz) : 0,86(3H,d,J = 6,5); 1,23(6H,t,J = 7); 2,14(3H,s); 3,52 et 3,68(4H,m); 4,21(1H,d,J = 6,5); 5,04 et 5,35(2H,2s); 7,12 et 7,38(4H,2d,J = 8) δ ppm.

## Example 4

N-{3-[4-(1-Méthoxy-1-méthyléthyl)-1-phényl]-2-méthylpropyl}-2,6-diméthylmorpholine

0,63 mole de diméthylmorpholine ont été ajoutés à température ambiante à une solution de 0,48 mole de 3-[-4-(1-méthoxy-1-méthyléthyl)-1-phényl]-2-méthylpropanal dans 100 ml de toluène placés dans un réacteur équipé d'un séparateur de type Dean-Stark. La réaction est légèrement exothermique. Le mélange a été ensuite chauffé à reflux pendant 4 h puis refroidi à température ambiante. La solution résultante a été directement transférée dans un réacteur d'hydrogénation et soumise à hydrogénation à pression atmosphérique en présence de 5 g de palladium à 5% sur charbon. Après absorption d'un équivalent d'hydrogène, le catalyseur a été filtré et le toluène a été évaporé, puis le résidu obtenu a été distillé à pression réduite. Le produit désiré a été obtenu avec un rendement de 75%.

| | |
|---|---|
| Eb. | 112°/0,1 mbar. |
| IR : | 3000-2600, 190-1590 $cm^{-1}$; |
| SM : | m/z : 128(100), 43(8), 70(8), 129(8), 159(2), 84(2), 55(1), 145(1), 319(1); |
| $^1$H RMN(360MHz) : | 0,85(3H,d,J = 7); 1,15$^{1)}$ et 1,23/1,26$^{2)}$ (6H,d,J = 6,8); 1,52(6H,s); 1,68(2H,q,J = 10); 1,9-2,45(5H,m); 2,63-2,82(2H,m); 3,06(3H,s); 3,68$^{1)}$ and 4,2$^{2)}$ (2H,m); 7,12 et 7,31-(4H,AB,J = 7,2) δ ppm. |

1) isomère cis 2) isomère trans

En suivant la même procédure que celle décrite ci-dessus, les composés suivants ont été obtenus :

N-{3-[4-(1-méthoxy-1-méthyléthyl)-1-phényl]-2-méthylpropyl)-morpholine :

| | |
|---|---|
| Eb. | 115°/0,1 mbar. |
| IR : | 3100-2700,1900-1590 $cm^{-1}$; |
| SM : | $M^+$ = 291(0,5) ; m/z : 100(100), 56(5), 91(2), 70(2), 43(2), 115(1), 161(1), 173(1); |
| $^1$H RMN(360MHz) : | 0,85(3H,d,J = 7); 1,52(6H,s); 1,97(1H,m); 2,16(2H,m); 2,26-2,45(5H,m); 2,80-(1H,dd,$^1$J = 12,5,$^2$J = 5,4); 3,06(3H,s); 3,70(4H,m); 7,11 et 7,30(4H,AB,J = 7,2) δ ppm. |

La morpholine a été utilisée à la place de la 2,6-diméthyl-morpholine. N-{3-[4-(1-méthoxy-1-méthyléthyl)-1-phényl]-2-méthylpropyl)-pipéridine :

| | |
|---|---|
| Eb. | 104°/0,1 mbar. |
| IR : | 3100-2700,1900-1550 $cm^{-1}$; |

SM : $M^+ = 289(1)$ ; m/z : 98(100), 99(5), 96(4), 55(3), 70(2), 41(2), 91(1);
$^1H$ RMN(360MHz) : 0,83(3H,d,J = 7); 1,42(2H,m); 1,52(6H,s); 1,57(4H,m); 1,97(1H,m); 2,13(2H,m); 2,23-2,38(5H,m); 2,81(1H,dd,$^1J = 12,6$,$^2J = 4,5$);
3,06(3H,s); 7,12 et 7,29(4H,AB,J = 7,2) $\delta$ ppm.
La pipéridine a été utilisée à la place de la 2,6-diméthyl-morpholine.

Example 5

N-[3-(4-isopropényl-1-phényl)-2-méthylpropyl]-pipéridine

0,5 Mole de pipéridine ont été ajoutées à une solution refroidie à 10° constituée par 87 g d'acide formique à 98% placée dans un ballon tricol de 0,5 l équipé d'une colonne de distillation et maintenue sous courant d'azote.
La vitesse d'addition était telle que la température ne dépassait pas 15°. Le mélange de réaction a été ensuite chauffé à 70° et 0,45 mole de 3-[4-(1-méthoxy-1-méthyléthyl)-1-phényl]-2-méthylpropanal ont été ajoutées au mélange sous agitation. Du $CO_2$ se dégageait lentement pendant l'addition. Le mélange a ensuite été chauffé à 100° pendant 2 h pendant que le méthanol distillait partiellement. L'excès d'acide formique a été distillé à 100° sous pression réduite, puis 76 g d'une solution de NaOH à 40% ont été ajoutés. Après refroidissement, 50 ml de toluène ont été ajoutés au mélange et la phase organique a été séparée. Après lavage avec de l'eau, le toluène a été évaporé et le résidu distillé. Le produit désiré a été obtenu avec un rendement d'environ 80%.
Eb. 93°/0,1 mbar.
IR : 3040, 3000-2600, 1900-1600, 1610 $cm^{-1}$;
SM : $M^+ = 257(1)$; m/z : 98(100),96(6), 55(4), 91(3), 70(3), 41(3), 131(2);
$^1H$ RMN(360MHz) : 0,84(3H,d,J = 7); 1,43(2H,m); 1,58(4H,m); 1,97(1H,m); 2,05-2,21(2H,m); 2,15(3H,s); 2,25-2,41(5H,m); 2,81(1H,dd,$^1J = 12,5$, $^2J = 5,2$); 5,04(1H,s); 5,36(1H,s); 7,12-(2H,d,J = 7,9) et 7,38(2H,d,J = 7,9) $\delta$ ppm.
En suivant la même procédure mais en utilisant de la morpholine à la place de la pipéridine, on a obtenu la N-[-3-(4-isopropényl-1-phényl)-2-méthylpropyl]-morpholine.
B.p. 103°/0,1 mbar.
IR : 3040, 3000-2600, 1900-1600, 1620 $cm^{-1}$;
SM : $M^+ = 259(1)$; m/z : 100(100), 56(6), 101(5), 91(4), 115(3), 131(2), 70(2);
$^1H$ RMN(360MHz) : 0,85(3H,d,J = 7); 1,96(1H,m); 2,15(3H,s); 2,08-2,23(2H,m); 2,28-2,46(5H,m); 2,80-(1H,dd,$^1J = 12,5$,$^2J = 5,8$); 3,71(4H,m); 5,04(1H,s); 5,35(1H,s); 7,11(2H,d,J = 7,6); 7,38(2H,d,J = 7,6) $\delta$ ppm.

Exemples d'application

Exemple 6

Une composition parfumante de type floral cyclamen a été préparée en mélangeant les ingrédients suivants (parties en poids) :

| | |
|---|---|
| Alcool cinnamique | 800 |
| Alcool phényléthylique | 900 |
| $\alpha$-Ionone | 800 |
| Acétate de benzyle | 500 |
| Rose absolue | 200 |
| Aldéhyde amylcinnamique | 200 |
| Jasmin absolue | 100 |
| Citral | 100 |
| 1-Citronellol | 1900 |
| Héliotropine | 700 |
| Musc cétone | 300 |
| Linalol | 500 |
| | 7000 |

A l'aide de la composition de base ci-dessus, on a préparé les mélanges que voici :

| Ingrédient | A | B | C |
|---|---|---|---|
| Base | 700 | 700 | 700 |
| Hydroxycitronellal synth. | 250 | - | 250 |
| Aldéhyde cyclamen | 50 | - | - |
| (a) | - | 250 | - |
| (b) | - | 50 | 50 |
| Total | 1000 | 1000 | 1000 |

(a) 3-[4-(1-méthoxy-1-méthyléthyl)-1-phényl]-2-méthylpropanal
(b) 3-(4-isopropényl-1-phényl)-2-méthylpropanal

Les mélanges ainsi obtenus ont été soumis à une évaluation comparative de la part d'un groupe d'experts qui se sont prononcés sur leur valeur odorante respective. De l'avis unanime, la composition B est celle qui possède la note florale la plus prononcée et qui montre le plus de volume. L'évaluation de la composition B montre également que le composé (a) peut valablement remplacer l'hydroxycitronellal, toutefois la composition B possède plus d'arrondi, de volume et de richesse par rapport à C et ne possède pas l'âpreté de A.

Exemple 7

Une composition de base de type floral lilas a été préparée en mélangeant les ingrédients suivants (parties en poids) :

| | |
|---|---|
| Terpinéol | 4000 |
| Alcool phényléthylique | 1200 |
| Linalol | 2000 |
| Aldéhyde anisique | 400 |
| Phénylacétaldéhyde diméthylacétal | 400 |
| Alcool cinnamique | 800 |
| Indol 10%* | 200 |
| | 9000 |

* diéthylphtalate

A l'aide de la composition de base ci-dessus, on a préparé les mélanges que voici :

| Ingrédient | A | B | C | D |
|---|---|---|---|---|
| Base | 900 | 900 | 900 | 900 |
| Lilial (marque enregistrée) [1)] | 100 | - | - | - |
| Aldéhyde cyclamen | - | 50 | - | - |
| Dipropylène glycol | - | 50 | - | - |
| (a) | - | - | 100 | - |
| (b) | - | - | - | 100 |
| Total | 1000 | 1000 | 1000 | 1000 |

(a) 3-[4-(1-méthoxy-1-méthyléthyl)-1-phényl]-2-méthylpropanal

(b) 3-(4-isopropényl-1-phényl)-2-méthylpropanal

1) aldéhyde p-tert-butyl-α-méthyl-hydrocinnamique ; marque déposée de L. Givaudan

Comme dans l'exemple précédent, les mélanges obtenus ont été soumis à une évaluation comparative de la part d'un groupe d'experts qui se sont prononcés sur les caractères odorants de chacun d'entre eux. Il est apparu que la composition C possède le plus de volume, de douceur et de velouté. Grâce à la présence du composé (a), la composition C possède une note lilas blanc, tandis que la composition D développe une note lilas fraîche, légèrement verte.

Exemples 8 - 20

On a procédé au parfumage des articles mentionnés ci-après, dans les concentrations indiquées, par adjonction de 3-[4-(1-méthoxy-1-méthyléthyl)-1-phényl]-2-méthylpropanal.

| | | conc. | odeur/aspect [25 ° C] | odeur/aspect [40 ° C]* |
|---|---|---|---|---|
| 8. | Eau de toilette alcoolique (alcool 95 ° ) | 5,0% | S/N | S/N |
| 9. | Crème huile/eau | 0,4% | S/N | S/N |
| 10. | Crème eau/huile | 0,4% | S/N | S/N |
| 11. | Shampoing | 0,5% | S/N | S/N |
| 12. | Déo spray | 0,8% | S/N | S/N |
| 13. | Spray laque | 0,3% | S/N | S/N |
| 14. | Savon | 0,5% | S/N | S/C |
| 15. | Talc | 0,5% | S/N | S/C |
| 16. | Détergent en poudre | 0,2% | S/N | S/N |
| 17. | Poudre à récurer | 0,2% | S/N | S/N |
| 18. | Désodorisant roll-on | 0,5% | S/N | S/N |
| 19. | Eau oxygénée | 0,2% | S/N | A/N |
| 20. | Permanente | 0,5% | S/T | S/T |

Clé des abréviations :
S = stable
N = normal
A = acceptable
T = trouble
C = coloration

* 1 mois à la température indiquée

Les essais de parfumage et stabilité résumés dans le tableau ci-dessus ont montré que le 3-[4-(1-méthoxy-1-méthyléthyl)-1-phényl]-2-méthylpropanal convient parfaitement au parfumage d'articles de consommation variés et peut trouver de ce fait un emploi étendu en parfumerie.
Des résultats analogues ont été observés avec l'emploi des acétals correspondants.

**Revendications**

**1.** Aldéhydes aromatiques et leurs dérivés de formule

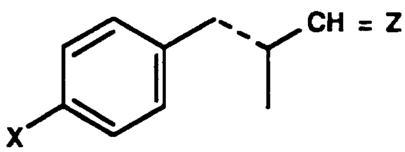

(I)

pouvant posséder une simple ou double liaison dans la position indiquée par les pointillés et dans laquelle le symbole X représente un radical monovalent de formule

a. 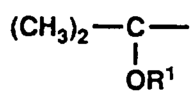

ou, lorsque les pointillés représentent une liaison simple, de formule

b. 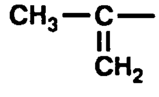

et le symbole Z sert à définir un atome d'oxygène ou deux restes $R^2O$, $R^1$ et $R^2$ représentant des

radicaux alkyle de 1 à 3 atomes de carbone.

2. Une aldéhyde aromatique ou un de ses dérivés suivant la revendication 1 choisie parmi les membres du groupe que voici :
3-[4-(1-méthoxy-1-méthyléthyl)-1-phényl]-2-méthylpropanal,
3-[4-(1-méthoxy-1-méthyléthyl)-1-phényl]-2-méthylpropionaldéhyde diméthylacétal,
3-[4-(1-méthoxy-1-méthyléthyl)-1-phényl]-2-méthylpropionaldéhyde diéthylacétal,
3-[-4-(1-méthoxy-1-méthyléthyl)-1-phényl]-2-méthyl-2-propénal,
3-(4-isopropényl-1-phényl)-2-méthylpropanal,
3-(4-isopropényl-1-phényl)-2-méthylpropionaldéhyde diméthylacétal, et
3-(4-isopropényl-1-phényl)-2-méthylpropionaldéhyde diéthylacétal.

3. Utilisation d'un aldéhyde aromatique ou d'un de ses dérivés selon la revendication 1 à titre d'ingrédient parfumant pour la préparation de compositions parfumantes et articles parfumés.

4. Composition parfumante résultant de l'utilisation selon la revendication 3.

5. Article parfumé résultant de l'utilisation selon la revendication 3.

6. A titre d'article parfumé selon la revendication 5, un savon, un cosmétique, un désodorisant d'air ambiant ou corporel, un détergent, un adoucissant textile ou un produit d'entretien.

7. Procédé pour la préparation d'un aldéhyde aromatique ou d'un de ses dérivés selon la revendication 1, caractérisé en ce qu'on
   a. additionne le propanal à l'aldéhyde 8-méthoxycuminique pour donner le 3-[4-(1-méthoxy-1-méthyléthyl)-1-phényl]-2-méthyl-2-propénal;
   b. réduit ledit composé ainsi formé par hydrogénation catalytique en présence d'un catalyseur métallique pour fournir le 3-[4-(1-méthoxy-1-méthyléthyl)-1-phényl]-2-méthylpropanal;
   c. traite le composé ainsi formé avec un agent acide fort pour fournir le 3-(4-isopropényl-1-phényl)-2-méthylpropanal et, le cas échéant,
   d. transforme les deux aldéhydes obtenus suivant les lettres b. ou c. ci-dessus en leurs dérivés acétals correspondants au moyen des méthodes usuelles.

8. Procédé selon la revendication 7, caractérisé en ce que le catalyseur métallique est le palladium sur un support d'alumine.

9. Procédé selon la revendication 7, caractérisé en ce que l'agent acide fort est l'acide sulfurique.

10. Composés hétérocycliques azotés de formule

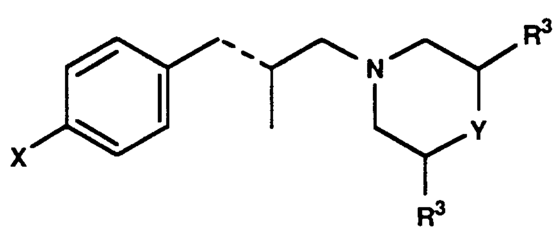

(II)

possédant une liaison simple ou double dans la position indiquée par la ligne pointillée et dans laquelle le symbole X représente un radical monovalent de formule

a. 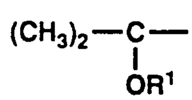

$R^1$ représentant un radical alkyle contenant de 1 à 3 atomes de carbone, ou, lorsque les pointillés

représentent une liaison simple, de formule

b. 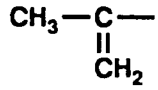

le symbole Y désigne un reste méthylène ou un atome d'oxygène et $R^3$ représente un atome d'hydrogène ou un groupe méthyle.

**11.** Un composé hétérocyclique azoté suivant la revendication 10 choisi parmi les composés que voici :
N-{3-[4-(1-méthoxy-1-méthyléthyl)-1-phényl]-2-méthylpropyl}-morpholine,
N-{3-[4-(1-méthoxy-1-méthyléthyl)-1-phényl]-2-méthylpropyl}-pipéridine,
N-{3-[4-(1-méthoxy-1-méthyléthyl)-1-phényl]-2-méthylpropyl}-2,6-diméthylmorpholine,
N-[-3-(4-isopropényl-1-phényl)-2-méthylpropyl]-pipéridine, et
N-[-3-(4-isopropényl-1-phényl)-2-méthylpropyl]-morpholine.

**12.** Utilisation d'un composé hétérocyclique azoté selon la revendication 10 à titre d'agent fongicide ou herbicide.

**Claims**

**1.** Aromatic aldehydes and derivatives thereof of general formula

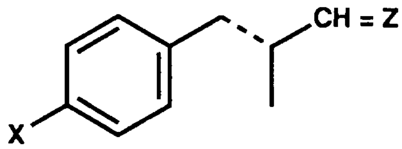

(I)

possessing a single or a double bond in the position indicated by the dotted line and wherein symbol X represents a monovalent radical or formula

a. 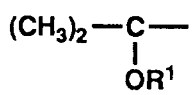

or, when the dotted line represents a single bond, of formula

b. 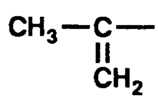

and wherein symbol Z stands for an oxygen atom or for two $R^2O$ radicals, $R^1$ and $R^2$ representing an alkyl radical having from 1 to 3 carbon atoms.

**2.** An aromatic aldehyde or a derivative thereof according to claim 1 selected amongst the members of the following group:
3-[4-(1-methoxy-1-methylethyl)-1-phenyl]-2-methylpropanal,
3-[4-(1-methoxy-1-methylethyl)-1-phenyl]-2-methylpropionaldehyde dimethylacetal,
3-[4-(1-methoxy-1-methylethyl)-1-phenyl]-2-methylpropionaldehyde diethylacetal,
3-[-4-(1-methoxy-1-methylethyl)-1-phenyl]-2-methyl-2-propenal,
3-(4-isopropenyl-1-phenyl)-2-methylpropanal,
3-(4-isopropenyl-1-phenyl)-2-methylpropionaldehyde dimethylacetal, and
3-(4-isopropenyl-1-phenyl)-2-methylpropionaldehyde diethylacetal.

3. Use of an aromatic aldehyde or a derivative thereof according to claim 1 as perfuming ingredient for the preparation of perfuming compositions and perfumed articles.

4. A perfuming composition resulting from the use according to claim 3.

5. A perfumed article resulting from the use according to claim 3.

6. As a perfumed article according to claim 5, a soap, a cosmetic, a body deodorant or an air-freshener, a detergent, a fabric softener or a household material.

7. Process for the preparation of an aromatic aldehyde or a derivative thereof according to claim 1 characterized in that:
   a. propanal is added to 8-methoxycuminic aldehyde to give 3-[4-(1-methoxy-1-methylethyl)-1-phenyl]-2-methyl-2-propenal;
   b. the thus formed compound is reduced by catalytic hydrogenation in the presence of a metal catalyst to give 3-[4-(1-methoxy-1-methylethyl)-1-phenyl]-2-methylpropanal;
   c. the resulting compound is treated with a strong acidic agent to give 3-(4-isopropenyl-1-phenyl)-2-methylpropanal, and, if desired,
   d. the two aldehydes obtained according to letter b. or c. above are converted into their corresponding acetal derivatives by means of current methods.

8. Process according to claim 7, characterized in that the metal catalyst is palladium on alumina.

9. Process according to claim 7, characterized in that the strong acidic agent is sulphuric acid.

10. Nitrogen heterocyclic compounds of formula

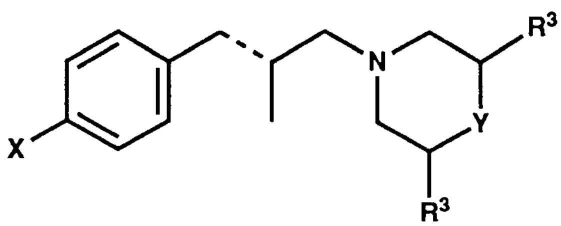

(II)

possessing a single or a double bond in the position indicated by the dotted line and wherein symbol X represents a monovalent radical or formula

a. 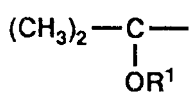

$R^1$ representing an alkyl radical having from 1 to 3 carbon atoms, or, when the dotted line represents a single bond, of formula

b. 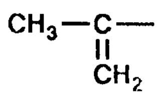

symbol Y represents a methylene radical or an oxygen atom and $R^3$ designates a hydrogen atom or a methyl radical.

11. A nitrogen heterocyclic compound according to claim 10, selected amongst the following compounds:
N-{3-[4-(1-methoxy-1-methylethyl)-1-phenyl]-2-methylpropyl}-morpholine,
N-{3-[4-(1-methoxy-1-methylethyl)-1-phenyl]-2-methylpropyl}-piperidine,

N-{3-[4-(1-methoxy-1-methylethyl)-1-phenyl]-2-methylpropyl}-2,6-dimethylmorpholine,
N-[-3-(4-isopropenyl-1-phenyl)-2-methylpropyl]-piperidine, and
N-[-3-(4-isopropenyl-1-phenyl)-2-methylpropyl]-morpholine.

**12.** Use of a nitrogen heterocyclic compound according to claim 10, as a fungicide or a herbicide agent.

**Patentansprüche**

**1.** Aromatische Aldehyde und ihre Derivate der Formel

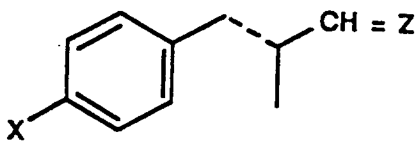

(I)

die eine Einfachbindung oder eine Doppelbindung in der durch die punktierten Linien angegebenen Stellung besitzen können und worin das Symbol X einen einwertigen Rest der Formel

a. 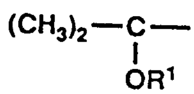

oder, falls die punktierten Linien eine Einfachbindung darstellen, der Formel

b. 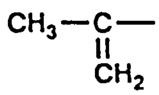

bedeutet und das Symbol Z dazu dient, ein Sauerstoffatom oder zwei Reste $R^2O$, $R^1$ und $R^2$ zu definieren, welche für Alkyl-Reste mit 1 bis 3 Kohlenstoffatomen stehen.

**2.** Ein aromatischer Aldehyd oder eines seiner Derivate gemäss Patentanspruch 1, ausgewählt unter den Gliedern der nachfolgenden Gruppe:
3-[4-(1-Methoxy-1-methylethyl)-1-phenyl]-2-methylpropanal,
3-[4-(1-Methoxy-1-methylethyl)-1-phenyl]-2-methylpropionaldehyd-dimethylacetal,
3-[4-(1-Methoxy-1-methylethyl)-1-phenyl]-2-methylpropionaldehyd-diethylacetal,
3-[4-(1-Methoxy-1-methylethyl)-1-phenyl]-2-methyl-2-propenal,
3-(4-Isopropenyl-1-phenyl)-2-methylpropanal,
3-(4-Isopropenyl-1-phenyl)-2-methylpropionaldehyd-dimethylacetal,
und
3-(4-Isopropenyl-1-phenyl)-2-methylpropionaldehyd-diethylacetal.

**3.** Verwendung eines aromatischen Aldehyds oder eines seiner Derivate gemäss Patentanspruch 1, als Riechstoffbestandteil für die Herstellung von Riechstoffzusammensetzungen und parfümierten Artikeln.

**4.** Riechstoffkomposition, resultierend aus der Verwendung gemäss Patentanspruch 3.

**5.** Parfümierter Artikel, resultierend aus der Verwendung gemäss Patentanspruch 3.

**6.** Eine Seife, ein Kosmetikum, ein Raumluftverbesserer oder ein Körperdesodorant, ein Reinigungsmittel, ein Textilauffrischungsmittel oder ein Pflegeprodukt als parfümierter Artikel gemäss Patentanspruch 5.

**7.** Verfahren zur Herstellung eines aromatischen Aldehyds oder eines seiner Derivate gemäss Patentanspruch 1, dadurch gekennzeichnet, dass man

a. das Propanal dem 8-Methoxycuminaldehyd zusetzt, um 3-[4-(1-Methoxy-1-methylethyl)-1-phenyl]-2-methyl-2-propenal zu ergeben;
b. die so gebildete Verbindung mittels katalytischer Hydrierung in Anwesenheit eines Metallkatalysators reduziert, um 3-[4-(1-Methoxy-1-methylethyl)-1-phenyl]-2-methylpropanal zu ergeben;
c. die so gebildete Verbindung mit einem stark saueren Mittel behandelt, um 3-(4-Isopropenyl-1-phenyl)-2-methylpropanal zu ergeben, und, gegebenenfalls,
d. die beiden, gemäss den obigen Stufen b. oder c. erhaltenen Aldehyde unter Verwendung üblicher Methoden in ihre entsprechenden Acetale überführt.

**8.** Verfahren gemäss Patentanspruch 7, dadurch gekennzeichnet, dass der Metallkatalysator Palladium auf einem Aluminiumoxid-Träger ist.

**9.** Verfahren gemäss Patentanspruch 7, dadurch gekennzeichnet, dass das stark sauere Mittel Schwefelsäure ist.

**10.** Heterocyclische Stickstoffverbindungen der Formel

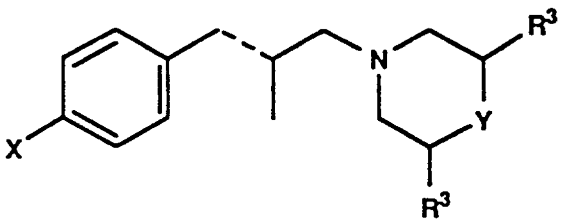

(II)

die eine Einfachbindung oder Doppelbindung an der durch die punktierte Linie angegebenen Stelle besitzen und worin das Symbol X einen einwertigen Rest der Formel

a. 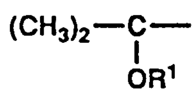

wobei $R^1$ für einen Alkylrest mit 1 bis 3 Kohlenstoffatomen steht,
oder, falls die punktierte Linie für eine Einfachbindung steht, der Formel

b. 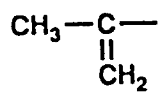

bedeutet, das Symbol Y einen Methylen-Rest oder ein Sauerstoffatom bedeutet und $R^3$ für ein Wasserstoffatom oder eine Methylgruppe steht.

**11.** Eine heterocyclische Stickstoffverbindung gemäss Patentanspruch 10, ausgewählt unter den nachfolgenden Verbindungen:
N-{3-[4-(1-Methoxy-1-methylethyl)-1-phenyl]-2-methylpropyl}-morpholin,
N-{3-[4-(1-Methoxy-1-methylethyl)-1-phenyl]-2-methylpropyl}-piperidin,
N-{3-[4-(1-Methoxy-1-methylethyl)-1-phenyl]-2-methylpropyl}-2,6-dimethylmorpholin,
N-[-3-(4-Isopropenyl-1-phenyl)-2-methylpropyl]-piperidin und
N-[-3-(4-Isopropenyl-1-phenyl)-2-methylpropyl]-morpholin.

**12.** Verwendung einer heterocyclischen Stickstoffverbindung gemäss Patentanspruch 10 als Fungizid oder Herbizid.